# EUROPEAN PATENT SPECIFICATION

(11) **EP 1 711 170 B1**
(45) Date of publication and mention of the grant of the patent: **29.07.2009**
(21) Application number: 05705748.1
(22) Date of filing: 14.01.2005
(51) Int. Cl.: A61K 9/70, A61K 31/455

(54) **TRANSDERMAL DELIVERY DEVICE FOR DIHYDROPYRIDINE TYPE CALCIUM ANTAGONISTS CONTAINING TWO FATTY ACIDS**
TRANSDERMALE ABGABEVORRICHTUNG FÜR DIHYDROPYRIDIN-ARTIGE CALCIUM-ANTAGONISTEN MIT 2 FETTSÄUREN
DISPOSITIF D'ADMINISTRATION TRANSDERMIQUE D'ANTAGONISTES CALCIQUES DE TYPE DIHYDROPYRIDINE RENFERMANT DEUX ACIDES GRAS

(30) Priority: 14.01.2004 US 536344 P
(43) Date of publication of application: 18.10.2006
(73) Proprietor: Lavipharm Laboratories, Inc., East Windsor, NJ 08536 (US)
(72) Inventor: OSBORNE, James, East Windsor, NJ 08536 (US); CHAVAN, Meenal, East Windsor, NJ 08536 (US)
(74) Representative: Agasse, Stéphane
(86) International application number: PCT/US2005/001302
(87) International publication number: WO 2005/067897

(56) References cited:
- EP-A- 0 296 553
- EP-A- 0 680 759
- WO-A-02/34206
- US-A- 5 391 377
- US-A- 5 854 281
- US-A- 5 925 373
- US-A1- 2002 028 234
- US-A1- 2002 160 995
- SQUILLANTE E ET AL: "Optimization of in vitro nifedipine penetration enhancement through hairless mouse skin" INTERNATIONAL JOURNAL OF PHARMACEUTICS 15 JUL 1998 NETHERLANDS, vol. 169, no. 2, 15 July 1998 (1998-07-15), pages 143-154, XP002331305 ISSN: 0378-5173
- SANTUS G C ET AL: "TRANSDERMAL ENHANCER PATENT LITERATURE" JOURNAL OF CONTROLLED RELEASE, ELSEVIER SCIENCE PUBLISHERS B.V. AMSTERDAM, NL, vol. 25, no. 1 / 2, 27 May 1993 (1993-05-27), pages 1-20, XP000361364 ISSN: 0168-3659

## Description

The present invention relates to formulations to deliver dihidropyridine-type calcium antagonists through the skin at a sufficient rate for treatment of hypertension for a duration up to one week.

The following prior art discloses different types of transdermal delivery devices:
Patent application EP 0296 553 disclosing a pharmaceutical composition for percutaneous drug absorption which comprises 5-isopropyl 3-methyl 2-cyano-6-methyl-4-(3-nitrophenyl)-1,4-dihydropyridine-3,5-dicarboxyla te,and ethanol and/or an unsaturated higher fatty acid;
Patent application EP 0 680 759 disclosing a composition for transdermal delivery of a dihydropyridine-type calcium antagonist such as nifedipine, nimodipine or nitrendipine comprises 1 to 20 weight % of the calcium antagonist in a mixed liquid comprising 0.1 to 50 mole % cis-oleic acid and 0.1 to 97 mole % dimethylisosorbide dispersed in a propylene glycol base;
   Scientific document SQUILLANTE E. ET AL. "Optimization of in vitro nifedipine penetration enhancement through hairless mouse skin" INTERNATIONAL JOURNAL OF PHARMACEUTICS 15 JUL 1998 NETHERLA NDS, vol 169, n° 2, 15 July 1998 (1998-07-15), pages 143-154, disclosing topical drug delivery system;
Patent application WO 2002/34206 disclosing a method of effectively treating hypertension in humans is achieved by administering felodipine via a transdermal formulation.

### Summary of the Invention

In one embodiment, the invention relates to a transdermal delivery device comprising a backing layer and an adhesive matrix reservoir. The backing layer may be opaque. The adhesive matrix reservoir is affixed to a side of the backing layer and is comprised of a dihydropyridine type calcium antagonist and oleic and linoleic acids permeation enhancer. In some embodiments, the adhesive matrix reservoir is essentially free of solvent. The present invention is suitable for light-sensitive drugs.

The fatty acid permeation enhancer should result in an increased aggregate transdermal delivery over 24 hours by 5%. In some preferred embodiments, the permeation enhancer increases aggregate transdermal delivery over 24 hours by at least 20 %. The adhesive matrix reservoir contains two distinct fatty acid permeation enhancers such as oleic and linoleic acids. The two distinct fatty acid permeation enhancers differ in degree of unsaturation or the chain length of the molecule.

The dihydropyridine type calcium antagonist may comprise at least 20%, or at least 50% or more by weight of the adhesive matrix reservoir.

In another aspect of the invention, the formulation may also include a stabilizer, which prevents degradation of the dihydropyridine type calcium antagonist. Suitable stabilizers include, but are not limited to, antioxidants such as BHT or BHA.

### Brief Description of the Drawings

FIG. 1 is a table of data which demonstrates suitable base polymers, drug ranges, polymer ranges and enhancers for the present invention.
FIG. 2A is a graphic representation of the permeation profile of dihydropyridine type calcium antagonist felodipine from a transdermal medical device containing no permeation enhancer.

FIG. 2B is a graphic representation of the permeation profile of felodipine from a transdermal medical device containing 3% linoleic acid and 6% oleic acid as permeation enhancers.
FIG. 2C is a graphic representation of the permeation profile of felodipine from a transdermal medical device containing 4% linoleic acid and 8% oleic acid as permeation enhancers.
FIG. 3-A illustrates permeation data for Example 6.
FIG 3-B illustrates permeation data for Example 7.
FIG 3-C illustrates permeation data for Example 8.
FIG 3-D illustrates permeation data for Example 9.
FIG 4-A illustrates permeation data for Example 10.
FIG 4-B illustrates permeation data for Example 11.
FIG 4-C illustrates permeation data for Example 12.
FIG 4-D illustrates permeation data for Example 13.
FIG 5-A illustrates permeation data for Example 14.
FIG 5-B illustrates permeation data for Example 15.
FIG 5-C illustrates permeation data for Example 16.
FIG 5-D illustrates permeation data for Example 17.
FIG 5-E illustrates permeation data for Example 18.

### Detailed Description of the Invention

### GLOSSARY

The following definitions are provided to facilitate an understanding of certain terms used frequently herein.

The term "administration period" means the time period during which the delivery device releases an active agent or combination of active agents to a subject.

The terms "drug " and "drug composition" as used interchangeably herein refer to dihydropyridine type calcium antagonists.

The term "drug reservoir" means a composition made to retain and release a drug for transdermal delivery, which composition is produced by combining a drug and a matrix material. The drug reservoir can be a drug reservoir composition, a solid drug reservoir layer, a solid drug reservoir adhesive layer, or a liquid drug reservoir layer. In some embodiments, a drug reservoir can be a solid drug reservoir layer in a multilaminate transdermal drug delivery medical device. When combined with an adhesive, the drug reservoir can also be a solid drug reservoir adhesive layer, which can be used, for example, in a monolith trandermal drug delivery medical device. The drug reservoir can also comprise permeation enhancers, plasticizers, and any other suitable additive, unless otherwise noted.

The term "drug transferring effective relationship" means that a device adapted for transdermal administration of a drug is kept in sufficient contact with the skin of a subject to allow for transdermal entry of the drug.

The terms "effective amount" and "therapeutically effective amount" mean a nontoxic but sufficient amount of a compound to provide the desired local or systemic therapeutic effect.

The term "flux" means the in vitro rate of delivery of drug per unit area through an area of human cadaver skin at 32 degrees C.

The term "monolith" means a transdermal medical device wherein the drug reservoir layer comprises a drug for transdermal administration and an adhesive composition that maintains the medical device in transdermal drug administration permitting contact with the skin. In some embodiments, the monolith is a drug reservoir adhesive layer comprising a drug composition, an adhesive composition and a matrix composition.

The term "multilaminate" refers to a transdermal medical device that comprises at least two layers, including a drug reservoir layer. In some embodiments, the multilaminate transdermal medical device can comprise a solid drug reservoir layer, a rate controlling membrane layer, a backing layer and an adhesive layer.

The term "solid drug reservoir" means a drug reservoir that comprises less than 1% w/w of any solvent used in producing the drug reservoir composition, and preferably less than 1000 ppm. For example, in some embodiments, the solid drug reservoir produced using heptane and isopropyl alcohol comprises less than 1000 ppm heptane and less than 1000 ppm alcohol in the solid drug reservoir.

The term "permeation enhancer" means a natural or synthetic molecule which facilitates the absorption of a given active agent or combination of active agents through tissue.

The term "pressure-sensitive adhesive" refers to a viscoelastic material which adheres to substrates with the application of pressure and remains permanently tacky.

The term "subject" means an animal, preferably a mammal, more preferably a human.

The term "sustained release" means the continual release of an active agent or combination of active agents over a period of time.

"Thickness" unless otherwise indicated is measured in mils (a mil = one thousandth of an inch) and can be determined by measuring the spacing when a transdermal delivery medical device of the present invention is placed between two microscopic slides.

"Transdermal" or "percutaneous" delivery means delivery of a drug by passage into and through the skin, and/or other body surfaces as a portal for the administration of drugs by topical application of the drug thereto.

The term "solvent content" is the percent residual process solvent (e.g., water, heptane, isopropyl alcohol) per unit dose as measured to the Karl Fisher method (for water) or appropriate analytical techniques (such as gas chromatograph, and the like) and expressed in parts per million or as percent of the weight of a delivery device of the present invention.

Increases in "aggregate transdermal delivery" are measured in cadaver skin, with the aggregate amount determined directly or by integrating the area under the curve of a series of flux measurements. The increase is with respect to the same adhesive matrix reservoir composition, but without the fatty acid enhancer(s).

The "dihydropyridine type calcium antagonists" relevant to the current invention are those where the 2 and 6 positions of the dihydropyridine ring are substituted with methyl, the 3 and 5 positions are independently substituted with propyl, acetyl, methoxycarbonyl, ethoxycarbonyl or methoxymethoxycarbonyl (CH3OCH3OOC-), and the 4 position is substituted with a cyclopentyl or cyclohexyl ring that is substituted with 1 to 2 electron withdrawing groups selected from nitro or chloro. The term "dihydropyridine type calcium antagonist" encompasses pharmaceutically acceptable salts thereof, as well as the base form of the drug. In some embodiments, the dihydropyridine type calcium antagonist is felodipine or isradipine. Weight percentages herein refer to the compound normalized to the non-salt form.

"Fatty acid permeation enhancers" are oleic and linoleic acids that are effective to increase aggregate transdermal delivery of the respective dihydropyridine type calcium antagonist.

A "solvent" is a compound that in neat form is a liquid at 25°C. It is effective in solubilizing or suspending the formulation components. Typically, preferred solvents also are volatile so that residual solvent levels are easily reduced by moderate heat to a level less than 1% and preferably less than 1000 ppm.

### DETAILED DESCRIPTION

The present invention will now be further described through the following detailed description of the present invention, which detailed description is illustrative of the preferred embodiments of the present invention and is not intended to limit the scope of the invention as set forth in the appended claims. While the following detailed description describes the invention through reference to embodiments of the present invention utilizing dihydropyridine type calcium antagonists and analgesically effective derivatives thereof as the drug, it should be understood that other drugs are also suitable for use with the teachings of the present invention.

In one embodiment of the invention, compositions are provided that comprise an adhesive matrix material. Suitable adhesive matrices include, but are not limited to, Acrylate, PIB, silicone, and/or polyisobutylene. The adhesive matrix may also optionally include polydimethyl siloxane.

Backing materials are well known in the art and can comprise plastic films of polyethylene, vinyl acetate resins, ethylene/vinyl acetate copolymers, polyvinyl chloride, polyurethane, and the like, metal foils, non-woven fabric, cloth and commercially available laminates. The backing material generally has a thickness in the range of 2 to 1000 micrometers. In preferred embodiments, the backing material is substantially impermeable to the drug contained in the drug reservoir layer, as well as the other contents of the drug reservoir layer. The backing may be a multi-layer polymer film containing a layer of aluminum. For example, a backing material can comprise a multilaminate of polyethylene terephthalate (PET) and polyethylene-(vinyl acetate) (EVA) copolymer. Numerous examples of appropriate backing materials are recognized in the art. In some embodiments, the backing is opaque. Some non-limiting, specific examples of backing materials include: (1) a PET backing material with a sealable layer of EVA (e.g., 12% vinyl acetate, VA) coated on one side of the PET backing material; (2) a film comprising layers of low density PET, nylon, EVA, and ethylene vinyl alcohol; (3) a film comprising layers of low density polyethylene, nylon and EVA; (4) a bi-layer film comprising low density polythethylene and nylon; (5) a monolayer of polyethylene; or (6) a monolayer of PET.

Various suitable strippable release liners are also well known in the art and include a fluoropolymer (for example, fluorocarbon diacrylate) or silicone (polysiloxane polymers) coated polyester film produced at a nominal thickness of about 3 mils. Examples of suitable commercially available release liners include a 5 mil fluoropolymer coated polyester film manufactured by 3M (Minnesota, MN) sold as SCOTCHPAK 9742™. It is also possible to use films made of material other than polyester or polyethylene terephthalate (PET), with a fluoropolymer coating. For example, the film can also be made of polystyrene or polypropylene. The same materials can also be used with a different coatings such as silicone. For preferred embodiments in which a polysiloxane is part of the multiple polymeric adhesive system, the release liner must be compatible with the silicone adhesive. In certain preferred embodiments of the invention, a suitable commercially available liner is 3M's 1022 SCOTCH PAK™, a fluoropolymer coated polyester film produced at about 3 mils thickness.

Preferably, the medical devices of the invention comprise a solid drug reservoir wherein at least a portion, and preferably all of the peripheral edges remains unsealed. The term "peripheral edge" of the drug reservoir and backing layer refer to the areas around the edges, that would be sealed together to define a liquid or gel based drug reservoir. Unlike medical devices comprising a liquid or gel drug reservoir, the solid drug reservoirs of the medical devices of certain embodiments of the present invention need not be sealed around their peripheral edges. The peripheral edges of a liquid or gel drug reservoir layer should be substantially fluid-tight to prevent drug leakage from the reservoir through the seal between the backing layer and the membrane. In preferred embodiments, the medical devices of the invention that comprise a solid reservoir layer do not present a hazard of drug leakage if the patch is torn, and do not need to be sealed to ensure against leaking. Avoiding the need for sealing of the drug reservoir layer can potentially lower production costs by avoiding one or more additional industrial processing steps.

In some embodiments, the medical devices of the invention can further comprise a rate-controlling membrane. Rate controlling membranes are preferably 0.5 to 10 mils thick, preferably 1-5 mils thick, and can be comprised of, for example, low density polyethylene (LDPE), EVA copolymers (for example, with up to 40% w/w and preferably between about 5 and 19% w/w VA), heat sealable polyesters, elastomeric polyester block copolymers, PVC and the like.

In some embodiments, the rate controlling membrane can comprise a microporous or porous material. Microporous membranes have a distinct pore structure with pores ranging in diameter from approximately 0.08 to 0.5 microns, preferably from about 0.1 and 0.4 microns, and more preferably from about 0.2 and 0.4 microns. Examples of suitable microporous membranes include polyethylene and polypropylene films, nylon, and nitrocellulose film. Other embodiments of the present invention will utilize microporous polyethylene membranes, such as Celgard K-256, available from Hoechst-Celanese, Charlotte, N.C. Porous membranes have pores greater than about 3 microns in diameter. Such materials are available as woven and non-woven fabrics. These materials can also be fabricated from nylon, polypropylene, polyethylene, polyolefins and the like.

The configuration of the transdermal delivery systems of the present invention can be in any shape or size as is necessary or desirable. Preferred sizes of the patch are: from 5 to 60 cm². In order to deliver the drug at the required rate for the desired duration, the loading of the drug in the patch should be sufficient to maintain saturation of the drug. Illustratively, a single dosage unit may have a surface area in the range of 3.5 cm² to deliver approximately 0.5 mg of drug per day. In order to maintain saturation for seven days, the patch should contain approximately 3.5 mg of drug (7 days x 0.5 mg/day) in excess of the quantity needed to saturate the patch with drug. In the present invention, the solubility of the drug in the matrix has been measured between 20-30%. An acceptable coating weight for the adhesive/drug layer of the present invention is 5-10 mg/cm². For illustrative purposes, a patch of 3.5 cm² requires 4.4 mg of drug for saturation at a coating weight of 5 mg/cm², resulting in approximately 7.9 mg (45%) of drug required in the system. In another illustrative example, a patch of 3.5 cm² would require 8.8 mg of drug for saturation at a coating weight of 10 mg/cm², with an assumed solubility of 25%, with a total amount of drug in the system as 12.3 mg (35%). Similarly, 6.6 mg of drug is needed for saturation with a coating weight of 7.5 mg/cm² with 10.1 mg (39%) of total drug required in the system. As a result of the high solubility of drug in the acrylate adhesive matrix used for this product, relatively high drug concentrations are needed to maintain saturation of drug in the system and a constant rate of drug delivery for a duration of up to one week.

In some embodiments, the matrix compositions of the transdermal drug delivery system can, optionally, also contain agents known to accelerate the delivery of the drug through the skin. These agents have been referred to as skin-penetration enhancers, accelerants, adjuvants, and sorption promoters, and are collectively referred herein as "permeation enhancers." This class of agents includes those with diverse mechanisms of action including those which have the function of improving the solubility and diffusibility of the drug within the multiple polymer and those which improve percutaneous absorption, for example, by changing the ability of the stratum corneum to retain moisture, softening the skin, improving the skin's permeability, acting as penetration assistants or hair-follicle openers or changing the state of the skin including the boundary layer. Some of these agents have more than one mechanism of action, but in essence they serve to enhance the delivery of the drug.

The permeation enhancer should result in an increased aggregate transdermal delivery over 24 hours by 5%. In some embodiments, the permeation enhancer increases aggregate transdermal delivery over 24 hours by at least 20 %. In some embodiments, the adhesive matrix reservoir contain two distinct fatty acid permeation enhancers. The two distinct fatty acid permeation enhancers may differ in degree of unsaturation such as oleic and linoleic acids. The concentration of each enhancer should be between 0.5 to 20%.

The medical devices of the invention may further be provided with various thickeners, fillers and other additives known for use with dermal compositions. Where the composition tends to absorb water, for example, when lecithin is used as a co-solvent, hydrophilic fillers are especially useful. The present invention is particularly well-suited for light-sensitive drugs. Methods of the present invention may be easily performed under yellow light to further minimize degradation of active ingredient(s).

While some preferred device aspects of the invention are generally described above, the invention can be further illustrated with respect to certain selected embodiments illustrated in the Figures and presented below. The selected embodiments discussed below are included for illustrative purposes and are not intended, and should in no way be construed to, limit the scope of the generally described invention.

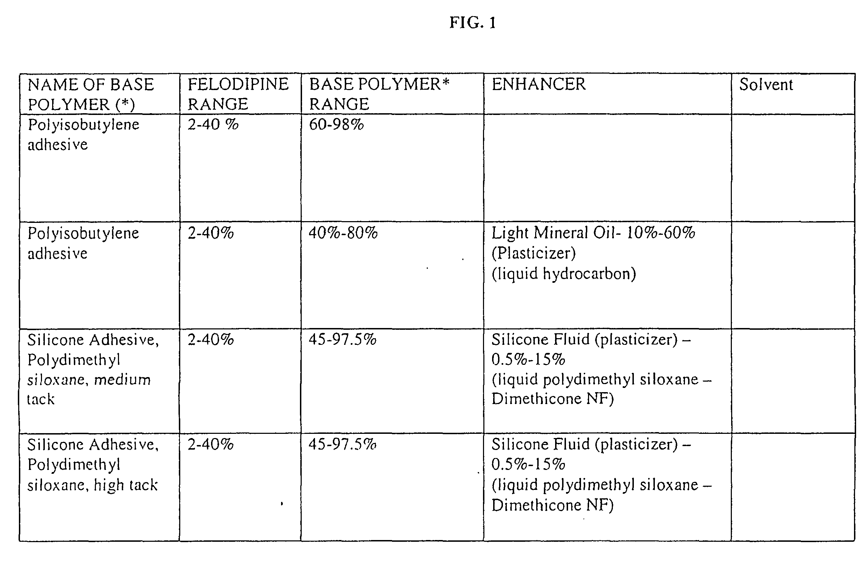

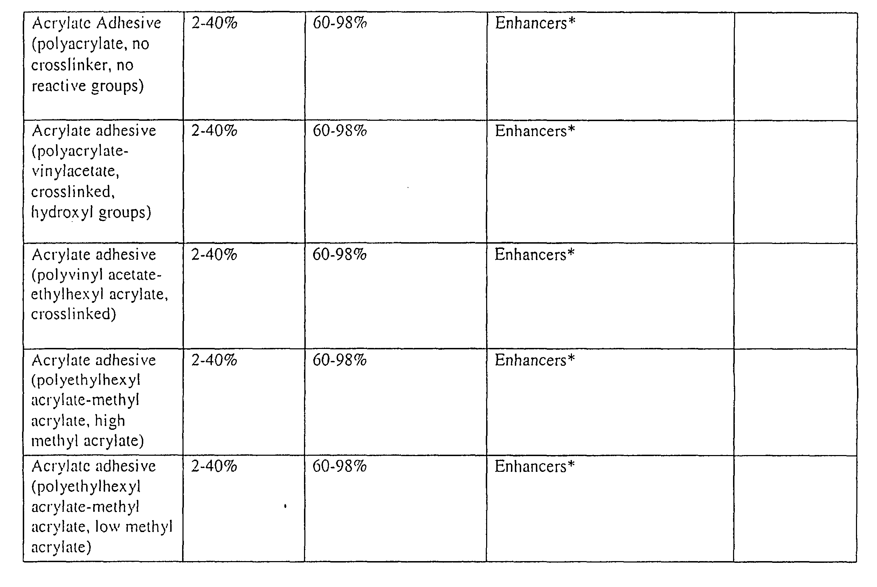

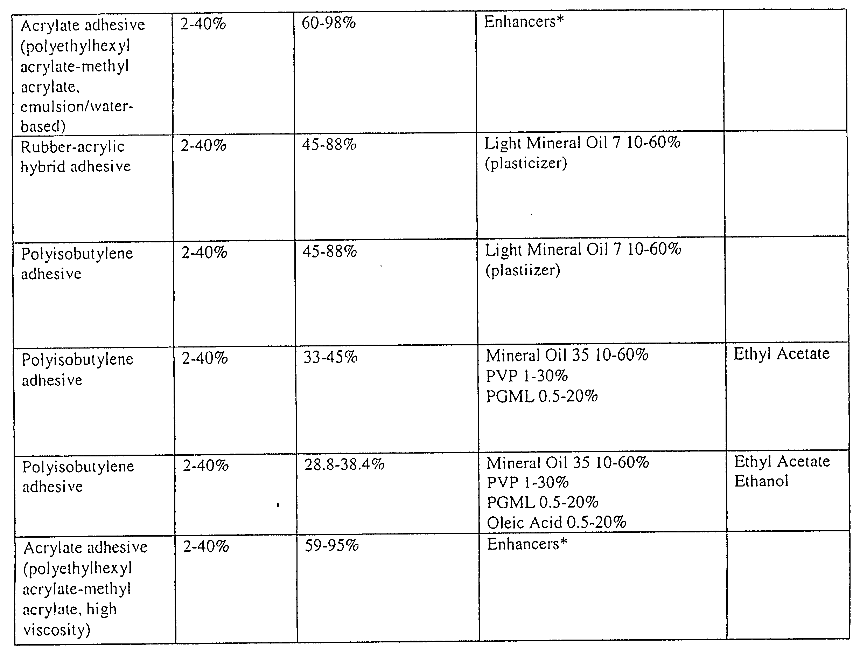

The data in **FIG. 1** demonstrates suitable base polymers, drug ranges, polymer ranges and enhancers for the present invention. Various base polymers (adhesives) suitable for the invention are provided in the Table, along with the range of suitable concentrations for the drug and for the base polymer. For each base polymer, a list of suitable enhancers is provided. Acrylate adhesives are compatible with the widest range of enhancers, and a list of enhancers that may be used with acrylate adhesives is provided separately within the Table. Acrylate adhesives are available with various functional groups, with and without crosslinkers. For this invention, acrylate adhesives without chemically reactive functional groups or crosslinkers are most suitable. Within these acrylate adhesives, oleic acid and linoleic acid are the most effective enhancers with the dihydropridine drugs. Some suitable solvents also are listed in the Table.

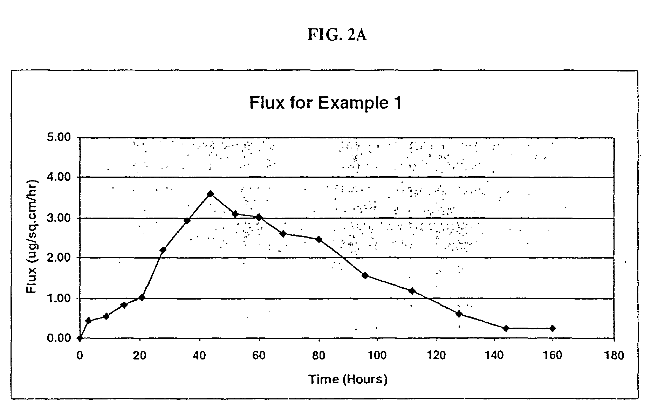

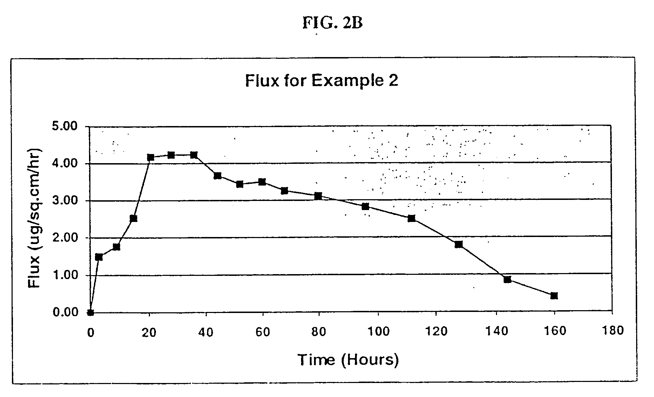

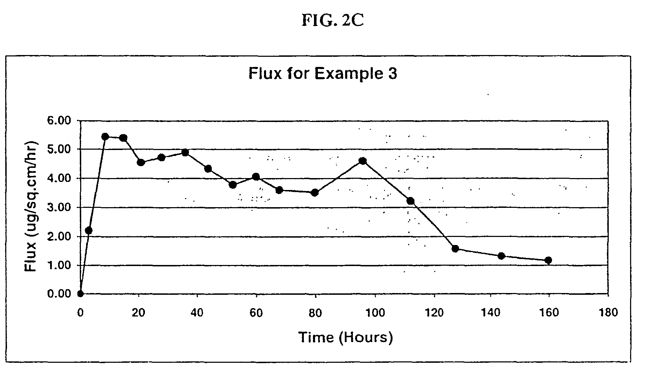

**FIG. 2** represents a series of graphs of the permeation profile of dihydropyridine type calcium antagonist felodipine from a transdermal medical device. Specifically, FIG. 2 provides a graphical representation of the dihydropyridine type calcium antagonists flux rate from the medical device, in µg/cm², as a function of time, in hours.

**FIG. 2-A** contains the in vitro flux data measured from a prototype formulation containing no permeation enhancer. The formulation consists of an adhesive reservoir attached to a backing, with the adhesive reservoir consisting of acrylate adhesive, felodipine, and a stabilizer, with no enhancer added. From this formulation, the flux in vitro measured over one week increased to a peak of about 3.5 µg/cm²-hr at about 3 days and decreased for the duration. The details regarding FIG. 2-A are provided in Example 1.

FIG. 2-B contains the in vitro flux data measured from a prototype formulation the same as the prototype represented in FIG. 2-A except the adhesive reservoir contains 3% linoleic acid and 6% oleic acid as permeation enhancers. From this formulation, the flux in vitro measured over one week increased to a peak of about 4.5 µg/cm²-hr at about 2.5 days and decreased for the duration.

FIG. 2-C contains the in vitro flux data measured from a prototype formulation the same as the prototype represented in FIG. 2-A except the adhesive reservoir contains 4% linoleic acid and 8% oleic acid as permeation enhancers. From this formulation, the flux in vitro measured over one week increased to a peak of about 5.5 µg/cm²-hr in less than 1 day and decreased for the duration. The series of graphs in FIG. 2 demonstrate that addition to the formulation of the permeation enhancers, linoleic acid and oleic acid, increases the in vitro flux rate and decreases the lag time to reach the maximum flux rate.

While many of the preferred embodiments of the invention are directed to dihydropyridine type calcium antagonists-containing transdermal delivery patches, the invention is not limited to patch devices. As appreciated by one skilled in the art, a variety of dihydropyridine type calcium antagonists-containing transdermal delivery devices can be made and used in accordance with the present invention. Such transdermal delivery devices are not limited to the form of the article and include, but are not limited to, articles such as a tape, a patch, a sheet, a dressing or any other form known to those skilled in the art. Generally the device will be in the form of a patch of a size suitable to deliver a pre-selected amount of dihydropyridine type calcium antagonists or other drug through the skin.

One skilled in the art given the above description of the compositions for solid drug reservoir layers or drug reservoir adhesive layers in various medical devices of the present invention will be able to produce those devices using a variety of known processing methods. Preferably, the compositions for making solid drug reservoir layers and drug reservoir adhesive layers of the present invention are produced using the following described process.

In preferred embodiments, the invention provides methods of treating disease by placing a dihydropyridine type calcium antagonist containing medical device in percutaneous drug flow permitting contact with an area of mammalian skin, preferably human skin. A dihydropyridine type calcium antagonists-containing device in accordance with this invention may be used to treat any condition capable of treatment with dihydropyridine type calcium antagonists, e.g., chronic and acute hypertension. The device can be placed on the skin and allowed to remain for an administration period sufficient to achieve or maintain the intended therapeutic effect. The time that constitutes a sufficient administration period can be selected by those skilled in the art with consideration of the flux rate of the device of the invention and of the condition being treated. In preferred embodiments, the medical devices of the present invention are maintained in drug flow permitting contact with an area of mammalian skin for a medically appropriate administration period.

Transdermal delivery of drugs offers a means of circumventing the problems of overdosing and under-dosing that are associated with conventional drug delivery methods. When a drug is administered intravenously or orally, the initial level of drug in the blood rapidly rises to a maximum, which is generally much higher than the therapeutically effective level of the drug. After the maximum level in the blood is reached, the concentration then falls slowly as the drug is distributed, metabolized, excreted, or degraded. Eventually, the blood concentration of the drug falls below the therapeutically effective level (i.e., there is "underdosing"). At this point, the drug needs to be re-administered to achieve effectiveness. Maintaining the blood concentration of drug between the minimum therapeutically effective level and toxic levels is important. One way to achieve this is to administer lower drug doses to the patient more frequently. This, however, is an unacceptable alternative in most instances, due to problems with patient compliance. The transdermal delivery of drugs can be designed so that the rate of delivery of the drug closely follows the rate of the clearance of the drug from the patient, thus keeping constant levels of drug in the blood, and reducing drug waste and overdosing problems.

In addition to the advantage of being able to control drug delivery rates, transdermal drug delivery also provides a comfortable, convenient and non-invasive method of administering drugs. Gastrointestinal irritation and other side-effects associated with oral drug delivery may be reduced or eliminated, and patient anxiety regarding invasive delivery methods, such as needles, is also eliminated.

### EXAMPLES

The following examples further illustrate the present invention, but of course, should not be construed as in any way limiting its scope.

### Example 1

Drug-Adhesive solution was prepared by mixing components on a wet basis as indicated in Table 1 to produce a formulation with dry composition 30.0% w/w of Felodipine, 69.5% w/w of Acrylate Adhesive (Gelva Multipolymer Solution 3071, Polyacrylate, no crosslinker, no reactive groups), and 0.5% w/w of BHT. Felodipine was dispersed in Ethyl Acetate by sonicating at 35°C for 30 minutes. Acrylate Adhesive Solution was added and the mixture was sonicated again at 35°C for 30 minutes. BHT was added and the mixture was rolled on a roller mill for about 24 hours to get a uniform dispersion. This coating solution was cast on the release liner (Medirelease 2249, siliconized polyester film) using the coater at a very low speed and using a suitable gauge to obtain a dry thickness of approximately 2 mils. The cast films were dried in the drying oven at 90°C for 20 minutes to remove the solvents. The backing film (Scotchpak 1109, multilayer polymer film with aluminum layer) was then laminated to the exposed surface of the dried cast adhesive on the release liner.

**Table 1 : Formulation for Example 1**

| **Ingredient** | **Wet Basis** **(% w/w)** | **Dry Basis** **(% w/w)** |
|---|---|---|
| Felodipine | 15.5 | 30.0 |
| Multipolymer Solution 3071 | 75.1 | 69.5 |
| BHT | 0.27 | 0.5 |
| Ethyl Acetate | 9.1 | 0.0 |

Permeation Procedure: The flux of Felodipine through human cadaver skin was measured using In-Line Auto Sampling Equipment. The experiment was run for 7 days using 40% aq. Ethanol solution as a receptor solution maintained at 32 ± 0.5°C. A circular patch of 0.62-cm² area was cut from the laminate; the release liner was removed, and the formulation mounted on a piece of skin (stratum corneum side) with the dried drug adhesive layer contacting the skin. The piece of skin along with patch was placed in the cell with the skin layer lying between the drug adhesive layer and receptor solution. At periodic time intervals, receptor solution flowing through the cell under the skin was collected and analyzed for Felodipine content by HPLC. Permeation Results for Example 1 are shown in Figure 2-A, which expresses flux in *µ*g/cm²/hr against time in hrs.

### Example 2

Drug-Adhesive solution was prepared by mixing components on a wet basis as indicated in Table 2 to produce a formulation with dry composition 30.0% w/w of Felodipine, 60.5% w/w of Acrylate Adhesive (Gelva Multipolymer Solution 3071, Polyacrylate, no crosslinker, no reactive groups), 3.0% of Linoleic acid (Crossential L98, Permeation enhancer), 6.0% of Oleic acid (Super Refined Oleic Acid, Permeation enhancer) and 0.5% w/w of BHT. Felodipine was dispersed in Ethyl Acetate by sonicating at 35°C for 30 minutes. Acrylate Adhesive Solution was added and the mixture was sonicated again at 35°C for 30 minutes. Linoleic acid, Oleic acid and BHT were added and the mixture was rolled on a roller mill for about 24 hours to get a uniform dispersion. This coating solution was cast on the release liner (Medirelease 2249, siliconized polyester film) using the coater at a very low speed and using a suitable gauge to obtain a dry thickness of approximately 2 mils. The cast films were dried in the drying oven at 90°C for 20 minutes to remove the solvents. The backing film (Scotchpak 1109, multilayer polymer film with aluminum layer) was then laminated to the exposed surface of the dried cast adhesive on the release liner.

**Table 2 : Formulation for Example 2**

| **Ingredient** | **Wet Basis** **(% w/w)** | **Dry Basis** **(% w/w)** |
|---|---|---|
| Felodipine | 16.5 | 30.0 |
| Multipolymer Solution 3071 | 69.2 | 60.5 |
| Linoleic acid | 1.6 | 3.0 |
| Oleic acid | 3.3 | 6.0 |
| BHT | 0.27 | 0.5 |
| Ethyl Acetate | 9.1 | 0.0 |

Permeation Procedure: The procedure for permeation experiment was followed as described in Example 1. Permeation Results for Example 2 are shown in Figure 2-B, which expresses flux in *µ*g/cm²/hr against time in hrs.

### Example 3

Drug-Adhesive solution was prepared by mixing components on a wet basis as indicated in Table 3 to produce a formulation with dry composition 30.0% w/w of Felodipine, 57.5% w/w of Acrylate Adhesive (Gelva Multipolymer Solution 3071, Polyacrylate, no crosslinker, no reactive groups), 4.0% of Linoleic acid (Crossential L98, Permeation enhancer), 8.0% of Oleic acid (Super Refined Oleic Acid, Permeation enhancer) and 0.5% w/w of BHT. The manufacturing process was same as described in Example 2.

**Table 3 : Formulation for Example 3**

| **Ingredient** | **Wet Basis** **(% w/w)** | **Dry Basis** **(% w/w)** |
|---|---|---|
| Felodipine | 16.8 | 30.0 |
| Multipolymer Solution 3071 | 67.1 | 57.5 |
| Linoleic acid | 2.3 | 4.0 |
| Oleic acid | 4.5 | 8.0 |
| BHT | 0.27 | 0.5 |
| Ethyl Acetate | 9.1 | 0.0 |

Permeation Procedure: The procedure for the permeation experiment was followed as described in Example 1. Permeation Results for Example 3 are shown in Figure 2-C, which expresses flux in *µ*g/cm²/hr against time in hrs.

### Example 4

Drug-Adhesive solution was prepared by mixing components on a wet basis as indicated in Table 4 to produce a formulation with dry composition 35.0% w/w of Felodipine and 65.0% w/w of Acrylate Adhesive (Duro-Tak® 87-4098, Polyacrylate-vinylacetate, crosslinked, hydroxyl groups). Felodipine was added to Acrylate Adhesive Solution and the mixture was rolled on a roller mill for about 24 hours to get a uniform dispersion. This coating solution was cast on the release liner (Medirelease 2249, siliconized polyester film) using the coater at a very low speed and using a suitable gauge to obtain a dry thickness of approximately 2 miL. The cast films were dried in the drying oven at 65°C for 20 minutes to remove the solvents. The backing film (Scotchpak 9733, polyester film laminate) was then laminated to the exposed surface of the dried cast adhesive on the release liner.

**Table 4 : Formulation for Example 4**

| **Ingredient** | **Wet Basis** **(% w/w)** | **Dry Basis** **(% w/w)** |
|---|---|---|
| Felodipine | 17.1 | 35.0 |
| Multipolymer Solution 3071 | 82.9 | 65.0 |

### Analysis of Felodipine

The formulations were analyzed for assay of Felodipine and presence of degradation products by HPLC. Time zero analysis showed presence of degradation product of Felodipine i.e. Felodipine Related Compound A. The formulations were stored at 40°C / 75% RH for 10 days to evaluate the effect on amount of degradation. An increase in the amount of degradant was observed after 10 days storage at 40°C / 75% RH. The results are expressed in Table 4-A.

**Table 4-A**

| | **% Content of** **Felodipine** | **Degradation Product** **(% w/w of active)** |
|---|---|---|
| Example 4 - Assay (Time zero) | 95.6 | 3.7 |
| Example 4 - Assay (40°C / 75% RH / 10 days) | 85.9 | 9.7 |

### Example 5

Drug-Adhesive solution was prepared by mixing components on a wet basis as indicated in Table 5 to produce a formulation with dry composition 30.0% w/w of Felodipine, 63.5% w/w of Acrylate Adhesive (Gelva Multipolymer Solution 3071, Polyacrylate, no crosslinker, no reactive groups), 2.0% of Linoleic acid (Crossential L98, Permeation enhancer), 4.0% of Oleic acid (Super Refined Oleic Acid, Permeation enhancer) and 0.5% w/w of BHT. The manufacturing process was same as described in Example 2.

**Table 5 : Formulation for Example 5**

| **Ingredient** | **Wet Basis** **(% w/w)** | **Dry Basis** **(% w/w)** |
|---|---|---|
| Felodipine | 16.2 | 30.0 |
| Multipolymer Solution 3071 | 71.5 | 63.5 |
| Linoleic acid | 1.1 | 2.0 |
| Oleic acid | 2.2 | 4.0 |
| BHT | 0.4 | 0.5 |
| Ethyl Acetate | 8.6 | 0.0 |

### Analysis of Felodipine

The formulations were analyzed for assay of Felodipine and presence of degradation products by HPLC. Time zero analysis showed no presence of degradation product of Felodipine i.e. Felodipine Related Compound A. The formulations were stored at 40°C / 75% RH for 12 weeks and were analyzed periodically to evaluate the effect on amount of degradation. No degradation was observed in samples stored up to 12 weeks at 40°C / 75% RH. These data demonstrate BHT is effective in preventing degradation of Felodipine to Felodipine Related Compound A.

### Examples 6, 7, 8 and 9

For Acrylate Adhesive (Multipolymer Solution with low Tg, Polyacrylate, no crosslinker, no reactive groups), four different formulations as Examples 6 to 9 were prepared containing 35% of drug and different ratios of enhancers. Drug-Adhesive solutions were prepared by mixing components on a wet basis as indicated in Tables 6 to 9 to produce the dry formulations specified in respective Tables 6 to 9. Felodipine was dispersed in Ethyl Acetate by mixing at a low speed using a propeller blade for 30 minutes. Acrylate Adhesive Solution was added and the mixture was mixed again at low speed for 30 minutes. Linoleic acid (Crossential L98, Permeation enhancer), Oleic acid (Super Refined Oleic Acid, permeation enhancer) and BHT (anti-oxidant) were added (required as per the formulation) and the mixture was mixed again at low speed for 30 minutes. The mixture was then rolled on a roller mill for about 24 hours to get a uniform dispersion. This coating solution was cast on the release liner (Medirelease 2249, siliconized polyester film) using the coater at a very low speed and using a suitable gauge to obtain a dry thickness of approximately 3 mils. The cast films were dried in the drying oven at 90°C for 20 minutes to remove the solvents. The backing film (Scotchpak 1109, multilayer polymer film with aluminum layer) was then laminated to the exposed surface of the dried cast adhesive on the release liner.

**Table 6 : Formulation for Example 6**

| **Ingredient** | **Wet Basis** **(% w/w)** | **Dry Basis** **(% w/w)** |
|---|---|---|
| Felodipine | 20.6 | 35.0 |
| Multipolymer Solution with low Tg | 79.4 | 64.9 |
| BHT | 0.1 | 0.1 |
| Ethyl Acetate | 10.0 | 0.0 |

**Table 7 : Formulation for Example 7**

| **Ingredient** | **Wet Basis** **(% w/w)** | **Dry Basis** **(% w/w)** |
|---|---|---|
| Felodipine | 20.9 | 35.0 |
| Multipolymer Solution with low Tg | 77.2 | 61.9 |
| Linoleic acid | 0.6 | 1.0 |
| Oleic acid | 1.2 | 2.0 |
| BHT | 0.1 | 0.1 |
| Ethyl Acetate | 10.0 | 0.0 |

**Table 8 : Formulation for Example 8**

| **Ingredient** | **Wet Basis** **(% w/w)** | **Dry Basis** **(% w/w)** |
|---|---|---|
| Felodipine | 21.4 | 35.0 |
| Multipolymer Solution with low Tg | 74.9 | 58.9 |
| Linoleic acid | 1.2 | 2.0 |
| Oleic acid | 2.4 | 4.0 |
| BHT | 0.1 | 0.1 |
| Ethyl Acetate | 10.0 | 0.0 |

**Table 9 : Formulation for Example 9**

| **Ingredient** | **Wet Basis** **(% w/w)** | **Dry Basis** **(% w/w)** |
|---|---|---|
| Felodipine | 22.2 | 35.0 |
| Multipolymer Solution with low Tg | 70.0 | 52.9 |
| Linoleic acid | 2.6 | 4.0 |
| Oleic acid | 5.1 | 8.0 |
| BHT | 0.1 | 0.1 |
| Ethyl Acetate | 10.0 | 0.0 |

### A. Permeation

Procedure: The flux of Felodipine through human cadaver skin was measured using In-Line Auto Sampling Equipment. The experiment was run for 7 days using 40% aq. Ethanol solution as a receptor solution maintained at 32 ± 0.5°C. A circular patch of 0.62-cm² area was cut from the laminate; the release liner was removed, and the formulation mounted on a piece of skin (stratum corneum side) with the dried drug adhesive layer contacting the skin. The piece of skin along with patch was placed in the cell with the skin layer lying between the drug adhesive layer and receptor solution. At periodic time intervals, receptor solution flowing through the cell under the skin was collected and analyzed for Felodipine content by HPLC.

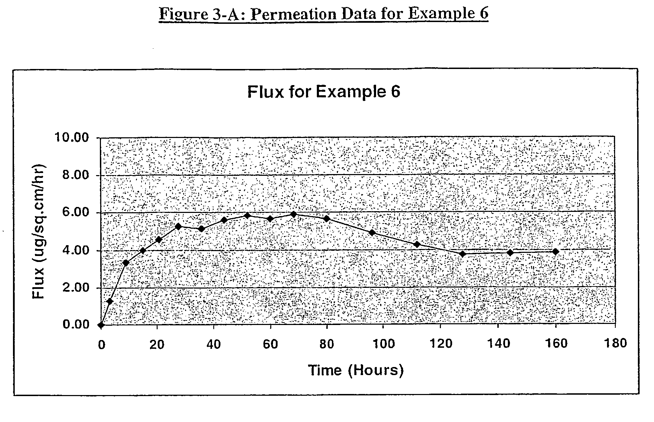

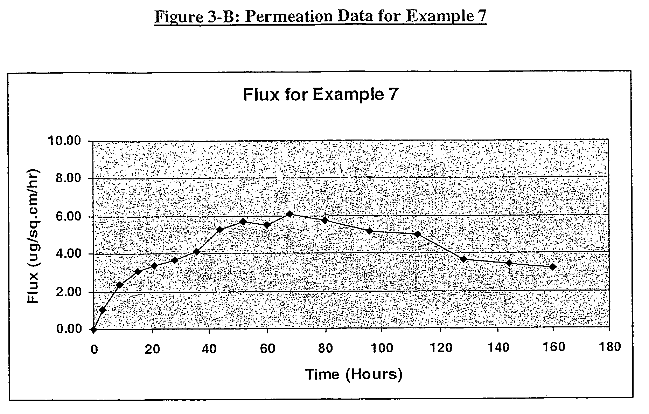

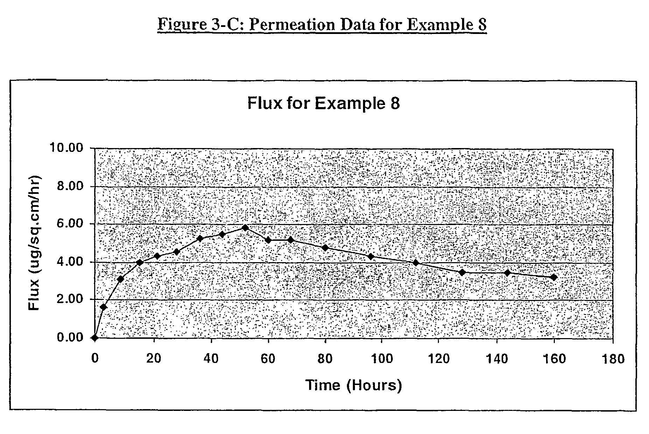

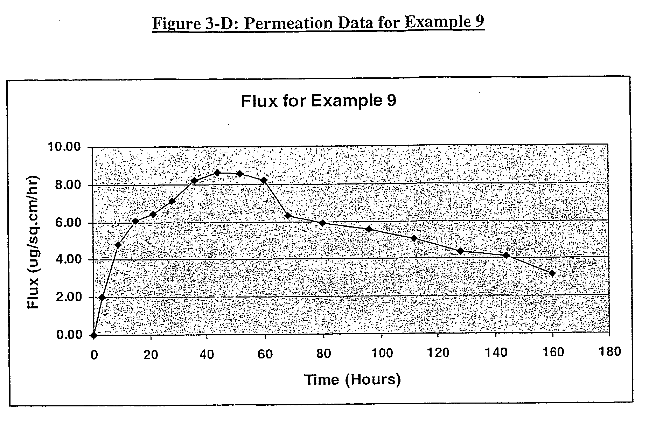

Permeation Results for Examples 6 to 9 are shown in Figures 3-A, 3-B, 3-C and 3-D, respectively, which expresses flux in *µ*g/cm²/hr against time in hrs.

### Examples 10,11, 12 and 13

For Acrylate Adhesive (Multipolymer Solution with high Tg, Polyacrylate, no crosslinker, no reactive groups), four different formulations as Examples 10 to 12 were prepared containing 35% of drug and different ratios of enhancers. Drug-Adhesive solutions were prepared by mixing components on a wet basis as indicated in Tables 10 to 12 to produce the dry formulations specified in respective Tables 10 to 12. The manufacturing procedure was followed as described in Examples 6, 7, 8 and 9.

**Table 10 : Formulation for Example 10**

| **Ingredient** | **Wet Basis** **(% w/w)** | **Dry Basis** **(% w/w)** |
|---|---|---|
| Felodipine | 17.4 | 35.0 |
| Multipolymer Solution with high T_{g} | 82.6 | 64.9 |
| BHT | 0.1 | 0.1 |
| Ethyl Acetate | 10.0 | 0.0 |

**Table 11 : Formulation for Example 11**

| **Ingredient** | **Wet Basis** **(% w/w)** | **Dry Basis** **(% w/w)** |
|---|---|---|
| Felodipine | 17.8 | 35.0 |
| Multipolymer Solution with high T_{g} | 80.6 | 61.9 |
| Linoleic acid | 0.5 | 1.0 |
| Oleic acid | 1.0 | 2.0 |
| BHT | 0.1 | 0.1 |
| Ethyl Acetate | 10.0 | 0.0 |

**Table 12 : Formulation for Example 12**

| **Ingredient** | **Wet Basis** **(% w/w)** | **Dry Basis** **(% w/w)** |
|---|---|---|
| Felodipine | 18.2 | 35.0 |
| Multipolymer Solution with high T_{g} | 78.6 | 58.9 |
| Linoleic acid | 1.1 | 2.0 |
| Oleic acid | 2.2 | 4.0 |
| BHT | 0.1 | 0.1 |
| Ethyl Acetate | 10.0 | 0.0 |

### B.

**Table 13 : Formulation for Example 13**

| **Ingredient** | **Wet Basis** **(% w/w)** | **Dry Basis** **(% w/w)** |
|---|---|---|
| Felodipine | 19.1 | 35.0 |
| Multipolymer Solution with high T_{g} | 74.2 | 52.9 |
| Linoleic acid | 2.2 | 4.0 |
| Oleic acid | 4.4 | 8.0 |
| BHT | 0.1 | 0.1 |
| Ethyl Acetate | 10.0 | 0.0 |

### C. Permeation

### Procedure: The procedure for permeation experiment was followed as described in Examples 6, 7, 8 and 9.

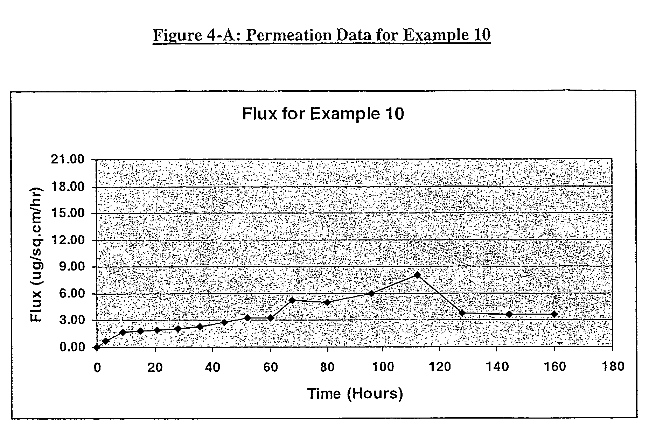

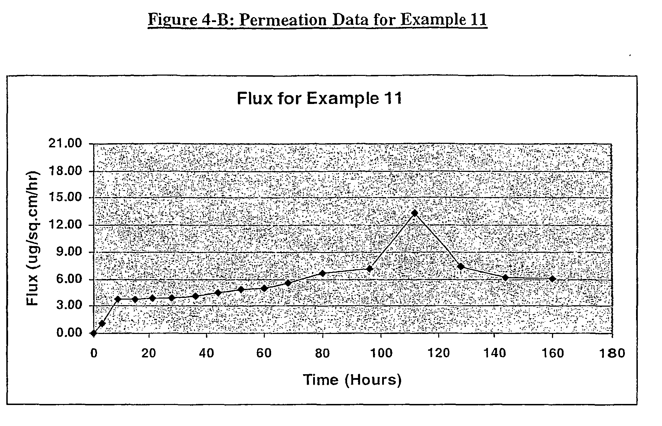

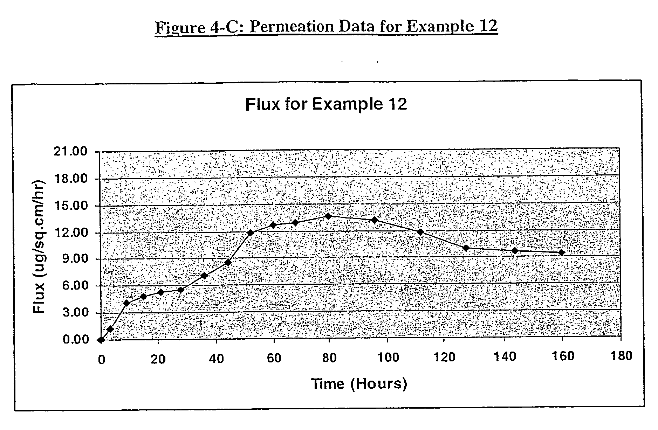

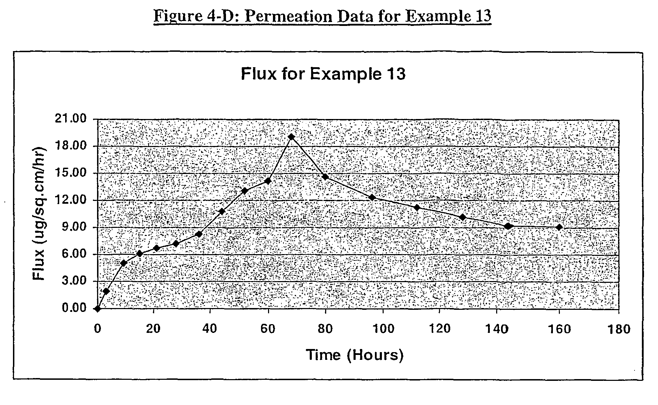

Permeation Results for Examples 10 to 13 are shown in Figures 4-A, 4-B, 4-C and 4-D, respectively, which expresses flux in *µ*g/cm²/hr against time in hrs.

### Examples 14, 15, 16, 17 and 18

For Acrylate Adhesive (Multipolymer Solution with middle Tg, Polyacrylate, no crosslinker, no reactive groups), five different formulations as Examples 14 to 18 were prepared containing 35% of drug and different ratios of enhancers. Drug-Adhesive solutions were prepared by mixing components on a wet basis as indicated in Tables 14 to 18 to produce the dry formulations specified in respective Tables 14 to 18. The manufacturing procedure was followed as described in Examples 6, 7, 8 and 9.

**Table 14 : Formulation for Example 14**

| **Ingredient** | **Wet Basis** **(% w/w)** | **Dry Basis** **(% w/w)** |
|---|---|---|
| Felodipine | 20.6 | 35.0 |
| Multipolymer Solution with middle Tg | 79.4 | 64.9 |
| BHT | 0.1 | 0.1 |
| Ethyl Acetate | 10.0 | 0.0 |

**Table 15 : Formulation for Example 15**

| **Ingredient** | **Wet Basis** **(% w/w)** | **Dry Basis** **(% w/w)** |
|---|---|---|
| Felodipine | 20.9 | 35.0 |
| Multipolymer Solution with middle Tg | 77.2 | 61.9 |
| Linoleic acid | 0.6 | 1.0 |
| Oleic acid | 1.2 | 2.0 |
| BHT | 0.1 | 0.1 |
| Ethyl Acetate | 10.0 | 0.0 |

**Table 16 : Formulation for Example 16**

| **Ingredient** | **Wet Basis** **(% w/w)** | **Dry Basis** **(% w/w)** |
|---|---|---|
| Felodipine | 21.4 | 35.0 |
| Multipolymer Solution with middle Tg | 74.9 | 58.9 |
| Linoleic acid | 1.2 | 2.0 |
| Oleic acid | 2.4 | 4.0 |
| BHT | 0.1 | 0.1 |
| Ethyl Acetate | 10.0 | 0.0 |

**Table 17 : Formulation for Example 17**

| **Ingredient** | **Wet Basis** **(% w/w)** | **Dry Basis** **(% w/w)** |
|---|---|---|
| Felodipine | 22.2 | 35.0 |
| Multipolymer Solution with middle Tg | 70.0 | 52.9 |
| Linoleic acid | 2.6 | 4.0 |
| Oleic acid | 5.1 | 8.0 |
| BHT | 0.1 | 0.1 |
| Ethyl Acetate | 10.0 | 0.0 |

**Table 18: Formulation for Example 18 (not the invention)**

| **Ingredient** | **Wet Basis** **(% w/w)** | **Dry Basis** **(% w/w)** |
|---|---|---|
| Felodipine | 18.7 | 35.0 |
| Multipolymer Solution with middle Tg | 69.8 | 62.8 |
| Linoleic acid | 1.1 | 2.0 |
| BHT | 0.1 | 0.2 |
| Ethyl Acetate | 10.4 | 0.0 |

### D. Permeation

### Procedure: The procedure for permeation experiment was followed as described in Examples 6, 7, 8 and 9.

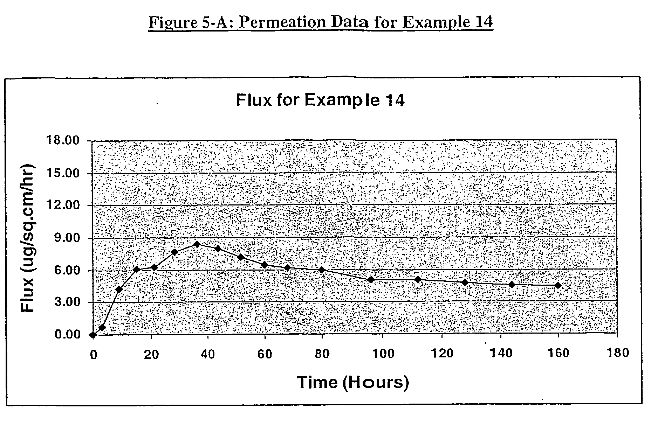

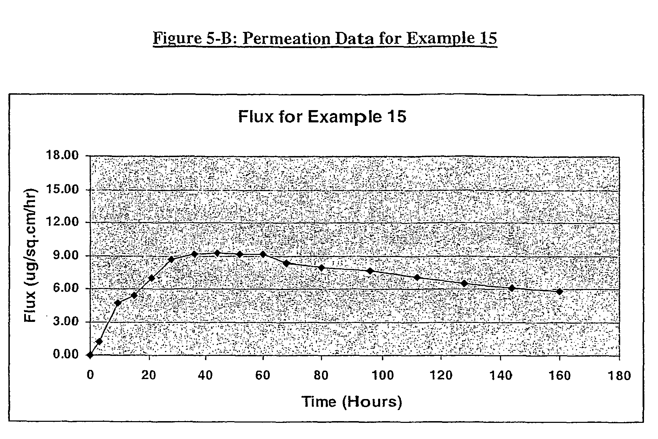

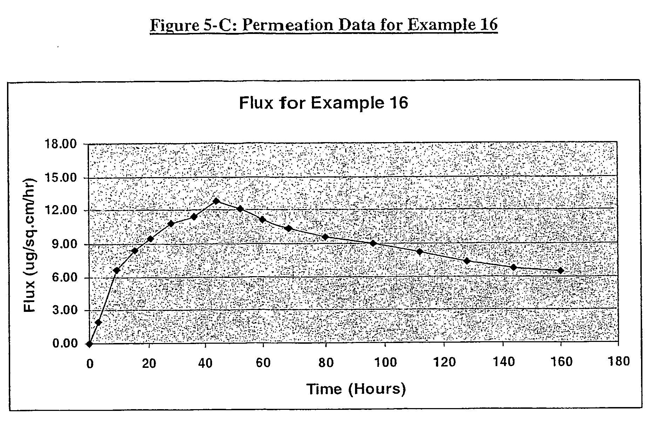

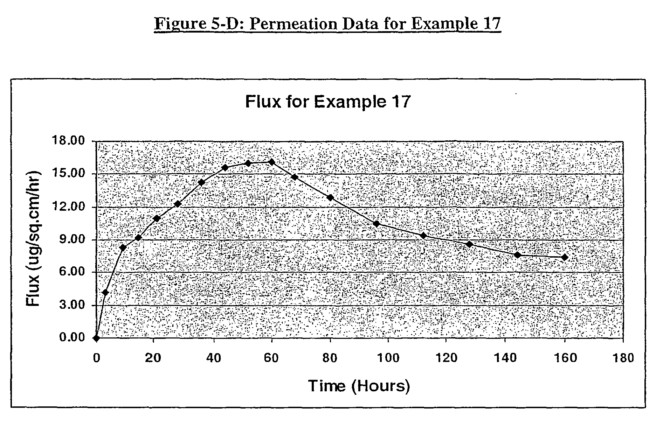

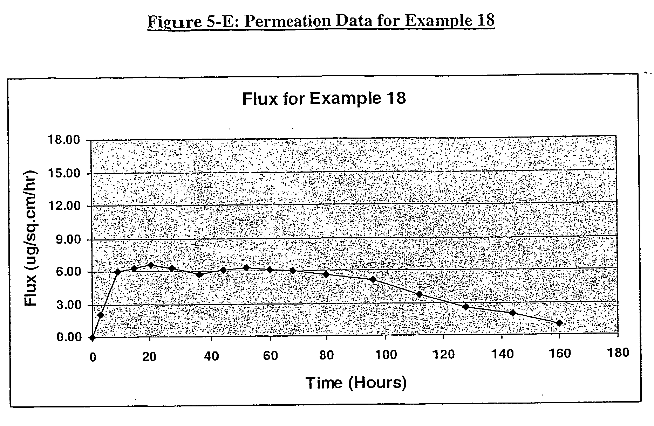

Permeation Results for Examples 14 to 18 are shown in Figures 5-A, 5-B, 5-C, 5-D and 5-E, respectively, which expresses flux in *µ*g/cm²/hr against time in hrs.

## Claims

1. A transdermal delivery device comprising : a backing layer; and an adhesive matrix reservoir affixed to a side of the backing layer, **characterized in that** the adhesive matrix reservoir comprises an adhesive matrix material, a dihydropyridine type calcium antagonist, and two fatty acid permeation enhancers, wherein the two fatty acid permeation enhancers comprise oleic and linoleic acids.

2. The transdermal delivery device of claim 1, wherein the fatty acid permeation enhancers are effective to increase aggregate transdermal delivery over 24 hours by 5%.

3. The transdermal delivery device of claim 2, wherein the fatty acid permeation enhancers are effective to increase aggregate transdermal delivery over 24 hours by at least 20%.

4. The transdermal delivery device of claim 1, wherein the adhesive matrix reservoir is essentially free of solvent.

5. The transdermal delivery device of claim 1, wherein the dihydropyridine type calcium antagonist comprises 20% or more by weight of the adhesive matrix reservoir.

6. The transdermal delivery device of claim 1, wherein the dihydropyridine type calcium antagonist comprises more than 20% by weight of the adhesive matrix reservoir.

7. The transdermal delivery device of claim 1, wherein the dihydropyridine type calcium antagonist comprises 25% or more by weight of the adhesive matrix reservoir.

8. The transdermal delivery device of claim 1, wherein the adhesive matrix material comprises at least 50% acrylate polymer.

9. The transdermal delivery device of claim 1, wherein the formulation further comprises a stabilizer, wherein said stabilizer prevents degradation of the dihydropyridine type calcium antagonist.

10. The transdermal delivery device of claim 9, wherein the stabilizer is BHT.

11. Thetransdeimal delivery device of claim 1, wherein the backing layer is opaque.

12. The transdermal delivery device of claim 1, wherein the dihydropyridine type calcium antagonist is felodipine.

13. The transdermal delivery device of claim 1 wherein the dihydropyridine type calcium antagonist is isradipine.

14. The transdermal delivery device of claim 1 wherein the oleic and linoleic acids are present in respective concentrations ranging from 0.5% to 20% (dry w/w).

15. The transdermal delivery device of claim 1 wherein the oleic and linoleic acids are present in respective concentrations selected from one of the following
1% (dry w/w) linoleic acid and 2% (dry w/w) oleic acid,
2% (dry w/w) linoleic acid and 4% (dry w/w) oleic acid
3% (dry w/w) linoleic acid and 6% (dry w/w) oleic acid, and
4% (dry w/w) linoleic acid and 8% (dry w/w) oleic acid.

## Patentansprüche

1. Transdermale Abgabevorrichtung, die Folgendes umfasst: eine Deckschicht und ein Klebstoffmatrix-Reservoir, das an einer Seite der Deckschicht befestigt ist, **dadurch gekennzeichnet, dass** das Klebstoffmatrix-Reservoir ein Klebstoffmatrixmaterial, einen Calciumantagonisten vom Dihydropyridin-Typ und zwei Fettsäure-Permeationsverstärker umfasst, worin die beiden Fettsäure-Permeationsverstärker Öl- und Linolsäure umfassen.

2. Transdermale Abgabevorrichtung nach Anspruch 1, worin die Fettsäure-Permeationsverstärker die gesamte transdermale Abgabe über 24 Stunden um 5% erhöhen.

3. Transdermale Abgabevorrichtung nach Anspruch 2, worin die Fettsäure-Permeationsverstärker die gesamte transdermale Abgabe über 24 Stunden um mindestens 20% erhöhen.

4. Transdermale Abgabevorrichtung nach Anspruch 1, worin das Klebstoffmatrix-Reservoir im Wesentlich frei von Lösungsmittel ist.

5. Transdermale Abgabevorrichtung nach Anspruch 1, worin der Calciumantagonist vom Dihydropyridin-Typ 20 Gew.-% oder mehr des Klebstoffmatrix-Reservoirs umfasst.

6. Transdermale Abgabevorrichtung nach Anspruch 1, worin der Calciumantagonist vom Dihydropyridin-Typ mehr als 20 Gew.-% des Klebstoffmatrix-Reservoirs umfasst.

7. Transdermale Abgabevorrichtung nach Anspruch 1, worin der Calciumantagonist vom Dihydropyridin-Typ 25 Gew.-% oder mehr des Klebstoffmatrix-Reservoirs umfasst.

8. Transdermale Abgabevorrichtung nach Anspruch 1, worin das Klebstoffmatrix-Material mindestens 50% Acrylatpolymer umfasst.

9. Transdermale Abgabevorrichtung nach Anspruch 1, worin die Formulierung ferner einen Stabilisator umfasst, worin der Stabilisator den Abbau des Calciumantagonisten vom Dihydropyridin-Typ verhindert.

10. Transdermale Abgabevorrichtung nach Anspruch 9, worin der Stabilisator BHT ist.

11. Transdermale Abgabevorrichtung nach Anspruch 1, worin die Deckschicht opak ist.

12. Transdermale Abgabevorrichtung nach Anspruch 1, worin der Calciumantagonist vom Dihydropyridin-Typ Felodipin ist.

13. Transdermale Abgabevorrichtung nach Anspruch 1, worin der Calciumantagonist vom Dihydropyridin-Typ Isradipin ist.

14. Transdermale Abgabevorrichtung nach Anspruch 1, worin die Öl- und Linolsäure jeweils in Konzentrationen von 0,5% bis 20% (auf Basis Trockengewicht) vorliegen.

15. Transdermale Abgabevorrichtung nach Anspruch 1, worin die Öl- und Linolsäure jeweils in einer der folgenden Konzentrationen vorliegen:
1% (Trockengewicht) Linolsäure und 2% (Trockengewicht) Ölsäure,
2% (Trockengewicht) Linolsäure und 4% (Trockengewicht) Ölsäure,
3% (Trockengewicht) Linolsäure und 6% (Trockengewicht) Ölsäure,
4% (Trockengewicht) Linolsäure und 8% (Trockengewicht) Ölsäure.

## Revendications

1. Dispositif d'administration transdermique comprenant : une couche de support ; et un réservoir à matrice adhésive fixé à un côté de la couche de support, **caractérisé en ce que** le réservoir à matrice adhésive comprend un matériau de matrice adhésive, un antagoniste calcique de type dihydropyridine, et deux promoteurs de perméation qui sont des acides gras, dans lequel les deux promoteurs de perméation qui sont des acides gras comprennent les acides oléique et linoléique.

2. Dispositif d'administration transdermique selon la revendication 1, dans lequel les promoteurs de perméation qui sont des acides gras sont efficaces pour augmenter de 5 % l'administration transdermique totale sur 24 heures.

3. Dispositif d'administration transdermique selon la revendication 2, dans lequel les promoteurs de perméation qui sont des acides gras sont efficaces pour augmenter d'au moins 20 % l'administration transdermique totale sur 24 heures.

4. Dispositif d'administration transdermique selon la revendication 1, dans lequel le réservoir à matrice adhésive est pratiquement exempt de solvant.

5. Dispositif d'administration transdermique selon la revendication 1, dans lequel l'antagoniste calcique de type dihydropyridine constitue 20 % ou plus en poids du réservoir à matrice adhésive.

6. Dispositif d'administration transdermique selon la revendication 1, dans lequel l'antagoniste calcique de type dihydropyridine constitue plus de 20 % en poids du réservoir à matrice adhésive.

7. Dispositif d'administration transdermique selon la revendication 1, dans lequel l'antagoniste calcique de type dihydropyridine constitue 25 % ou plus en poids du réservoir à matrice adhésive.

8. Dispositif d'administration transdermique selon la revendication 1, dans lequel le matériau de matrice adhésive comprend au moins 50 % de polymère acrylate.

9. Dispositif d'administration transdermique selon la revendication 1, dans lequel la formulation comprend en outre un stabilisateur, ledit stabilisateur prévenant la dégradation de l'antagoniste calcique de type dihydropyridine.

10. Dispositif d'administration transdermique selon la revendication 9, dans lequel le stabilisateur est le butylhydroxytoluène.

11. Dispositif d'administration transdermique selon la revendication 1, dans lequel la couche de support est opaque.

12. Dispositif d'administration transdermique selon la revendication 1, dans lequel l'antagoniste calcique de type dihydropyridine est la félodipine.

13. Dispositif d'administration transdermique selon la revendication 1, dans lequel l'antagoniste calcique de type dihydropyridine est l'isradipine.

14. Dispositif d'administration transdermique selon la revendication 1, dans lequel les acides oléique et linoléique sont présents dans des concentrations respectives de 0,5 % à 20 % (p/p sec).

15. Dispositif d'administration transdermique selon la revendication 1, dans lequel les acides oléique et linoléique sont présents dans des concentrations respectives sélectionnées parmi l'une des concentrations suivantes :
1 % (p/p sec) d'acide linoléique et 2 % (p/p sec) d'acide oléique,
2 % (p/p sec) d'acide linoléique et 4 % (p/p sec) d'acide oléique,
3 % (p/p sec) d'acide linoléique et 6 % (p/p sec) d'acide oléique, et
4 % (p/p sec) d'acide linoléique et 8 % (p/p sec) d'acide oléique.
